# EUROPÄISCHE PATENTSCHRIFT

(11) **EP 2 632 503 B1**
(45) Veröffentlichungstag und Bekanntmachung des Hinweises auf die Patenterteilung: **08.04.2015**
(21) Anmeldenummer: 11761361.2
(22) Anmeldetag: 26.09.2011
(51) Int. Cl.: A61L 27/30, A61L 27/56, C04B 41/87, C04B 41/89

(54) **KERAMIK-ENDOPROTHESE MIT KERAMISCHER BESCHICHTUNG UND VERFAHREN ZU DEREN HERSTELLUNG**
CERAMIC ENDOPROSTHESIS WITH CERAMIC COATING, AND METHOD FOR PRODUCING SAME
ENDOPROTHÈSE EN CÉRAMIQUE MUNIE D'UN REVÊTEMENT CÉRAMIQUE ET PROCÉDÉ DE PRODUCTION APPROPRIÉ

(30) Priorität: 11.03.2011 DE 102011005424; 29.10.2010 DE 102010050104
(43) Veröffentlichungstag der Anmeldung: 04.09.2013
(73) Patentinhaber: Mathys AG Bettlach, 2544 Bettlach (CH)
(72) Erfinder: GRÄTZ, Nadine, 37603 Holzminden (DE); OBERBACH, Thomas, 07629 Reichenbach (DE); BEGAND, Sabine, 99425 Weimar (DE); ORTMANN, Claudia, 07749 Jena (DE); DELFOSSE, Daniel, CH-3303 Jegenstorf (CH)
(74) Vertreter: Körfer, Thomas
(86) Internationale Anmeldenummer: PCT/EP2011/066649
(87) Internationale Veröffentlichungsnummer: WO 2012/055655

(56) Entgegenhaltungen:
- DATABASE WPI Week 199408 Thomson Scientific, London, GB; AN 1994-061509 XP002666994, & JP 6 014988 A (KYOCERA CORP) 25. Januar 1994 (1994-01-25)
- LI Z-L ET AL: "Bone-bonding behavior under load-bearing conditions of an alumina ceramic implant incorporating beads coated with glass-ceramic containing apatite and wollastonite", JOURNAL OF BIOMEDICAL MATERIALS RESEARCH, Bd. 29, Nr. 9, September 1995 (1995-09), Seiten 1081-1088, XP002666995, JOHN WILEY & SONS INC US DOI: DOI:10.1002/JBM.820290908
- KAKUTANI Y ET AL: "Strengthening of bone-implant interface by the use of granule coatings on alumina ceramics", JOURNAL OF BIOMEDICAL MATERIALS RESEARCH, Bd. 23, Nr. 7, Juli 1989 (1989-07), Seiten 781-808, XP002666996, US DOI: 10.1002/JBM.820230709
- PIZZOFERRATO A ET AL: "Multilayered bead ceramic composite coating for hip prostheses: Experimental studies and preliminary clinical results", JOURNAL OF BIOMEDICAL MATERIALS RESEARCH, Bd. 22, Nr. 12, Dezember 1988 (1988-12), Seiten 1181-1202, XP002666997, DOI: 10.1002/JBM.820221209

## Beschreibung

Die Erfindung bezieht sich auf eine Keramik-Endoprothese zur Implantation in einen tierischen oder menschlichen Knochen mit einer keramischen Beschichtung sowie ein Verfahren zu dessen Herstellung.

In der Endoprothetik werden Keramikimplantate häufig als Knochen- oder Gelenkersatz verwendet. Durch ihre hohe Bruchfestigkeit und Korrosionsbeständigkeit sowie ihr inertes Verhalten im Körper bei gleichzeitig hervorragendem Gleit- und Reibungsverhalten eignen sich Keramikimplantate insbesondere als Ersatz für Hüft- oder Schultergelenkpfannen und Schulter- oder Hüftköpfe sowie als Knieprothese. Bei einer zementfreien Befestigung des Implantats muss die mit dem Knochen in Kontakt stehende knochenseitige Oberfläche des Implantats aufgeraut oder strukturiert ausgebildet sein, um ein Einwachsen bzw. Verwachsen mit dem Knochen zu ermöglichen.

Zu diesem Zweck wird, wie in der DE 100 15 614 A1 beschrieben, z. B. auf eine Hüftgelenkpfanne aus Aluminiumoxid an ihrer knochenseitigen Oberfläche eine poröse Schicht ebenfalls aus Aluminiumoxid aufgetragen. Die Schicht wird aus dem für die Herstellung der Endoprothese vorgesehenen Werkstoff abgezweigt und mit porenbildenden Substanzen, wie z.B. kugel- oder linsenförmigen Teilchen aus Polyethylen-Wachs, Stärke oder Zellulose, versetzt. Die so hergestellte dickflüssige Suspension wird auf die knochenseitige Oberfläche der Hüftgelenkpfanne im Zustand des Grünkörpers aufgestrichen. Beim nachfolgenden Sintern werden die porenbildenden Teilchen verdampft oder verbrannt, sodass Poren zurückbleiben.

Ein Nachteil an dieser Ausführung ist es, dass in den vollständig von Werkstoff umschlossen Poren der Endoprothese Reste der porenbildenden Substanzen verbleiben und im Laufe der Zeit in das umgebende Knochenmaterial wandern. Dies kann zu einer Entzündung des Knochens und zu einer Lockerung der Endoprothese führen.

Es ist somit die Aufgabe der vorliegenden Erfindung, eine Keramik-Endoprothese mit einer porösen bzw. makrostrukturierten knochenseitigen Oberfläche sowie ein Verfahren zu deren Herstellung zu schaffen, deren poröse Keramikschicht weniger oder keine belastenden Rückstände von porenbildenden Substanzen aufweist.

Die Aufgabe wird durch die erfindungsgemäße Keramik-Endoprothese nach Anspruch 1 und durch das Verfahren zur Herstellung derselben gemäß Anspruch 9 gelöst. In den Unteransprüchen sind vorteilhafte Weiterbildungen der erfindungsgemäßen Keramik-Endoprothese bzw. des Verfahrens dargestellt.

Die erfindungsgemäße Keramik-Endoprothese zur Implantation in einen tierischen oder menschlichen Knochen umfasst einen Prothesenkörper, der eine mit dem Knochen in Kontakt stehende knochenseitige Oberfläche besitzt, die wiederum eine poröse Keramikschicht aus keramischem Granulat aufweist.

Das keramische Granulat wird bevorzugt als Suspension zu einem Schlicker vermengt und auf die Endoprothese aufgebracht. Durch Sintern wird das Granulat stoff- und/oder formschlüssig mit der Endoprothese verbunden.

Eine Keramik-Endoprothese mit einer porösen Keramikschicht aus keramischem Granulat hat den Vorteil, dass die Poren durch die Zwischenräume zwischen dem keramischen Granulat gebildet werden. Es wird keine porenbildende Substanz, wie z.B. Wachs, Stärke oder Zellstoff, verwendet, die als Platzhalter für die Poren dient und durch Ausbrennen oder Ausdampfen letztendliche Poren zurücklassen. Das Granulat selbst besteht aus keramischem Material und verursacht somit keine schädlichen Rückstände. Die in der Suspension bzw. dem Schlicker enthaltenen Binder und Additive sind nicht wie beim Stand der Technik vom Werkstoff vollständig umgeben, sondern umgeben die Granulatkörner. Sie können durch die Zwischenräume zwischen dem Granulat leicht entweichen, sodass keine Rückstände zurückbleiben.

In vorteilhafter Weise besteht die poröse Keramikschicht aus mehreren Granulat-Schichten mit unterschiedlichen Korngrößen. Somit kann eine gezielte Strukturierung der Keramikschicht mit größeren Korngrößen direkt an der knochenseitigen Oberfläche der Endoprothese hin zu Schichten mit kleinerer Korngröße zur Ausbildung von feinen Spitzen verwendet werden.

Von Vorteil ist es ebenfalls, wenn das Granulat selbst durch Sprühgranulierung hergestellt ist. Bei der Sprühgranulierung wird eine Keramiksuspension durch eine Düse in einem Sprühturm in kleine Tröpfchen zerstäubt. Granulatgröße und Form sind über eine Variation der Düsenöffnung und Düsenform, der Schlickerzusammensetzung und der Sprühparameter, wie z.B. Sprühdruck und Temperatur im Sprühturm, steuerbar. Während des Aufenthaltes der Tröpfchen im Sprühturm erfolgt deren Trocknung, so dass resultierend ein nahezu wasserfreies, kugelförmiges, poröses Granulat vorliegt. Ebenso ist die Herstellung des Granulats durch Aufbaugranulierung möglich.

Somit können sehr variable Korngrößen parallel zum Herstellungsprozess der Keramik-Endoprothese gefertigt werden. Alternativ zum Granulat können Keramikkügelchen, wie sie z.B. als Mahlkörper zum Zerkleinern von Keramikpulver in Mühlen, sogenannte Mahlkugeln, eingesetzt werden, verwendet werden. Diese sind kommerziell in verschiedenen Größen und Materialien erhältlich.

Um die Rauheit der Keramikschicht zu erhöhen, ist es von Vorteil, wenn das Granulat durch Mörsern oder Mahlen nochmals zerkleinert ist. Damit wird die glatte Oberfläche der Granulatkörner zerstört und durch Granulatfragmente Spitzen ausgeformt sowie eine raue Oberfläche erzeugt. So kann die poröse Keramikschicht aus einer nicht gemörserten oder gemahlene ersten Granulatschicht, auf der eine zweite Granulatschicht, die aus derart nachbearbeitetem Granulat besteht, aufgebaut sein.

Bei dem entsprechenden Verfahren zur Herstellung der vorgehend beschriebenen Keramik-Endoprothese wird eine poröse und/oder makrostrukturierte Keramikschicht aus keramischem Granulat auf die knochenseitige Oberfläche eines keramischen Prothesenkörpers aufgebracht.

Die poröse und/oder makrostrukturierte Keramikschicht besteht bevorzugt aus einem Granulat, das als Suspension zu einem Schlicker vermengt wird und auf den Prothesenkörper aufgebracht wird.

Vorteilhafterweise liegt der Prothesenkörper in ungebranntem oder vorgebranntem Zustand vor und weist damit auch eine Porosität auf. Durch die Porosität des Prothesenkörpers wird dem Schlicker durch Kapillarwirkung Lösungsmittel, wie z.B. Wasser entzogen, was die Haftung des Schlickers und des Granulates auf dem Prothesenkörper verbessert. Der Prothesenkörper kann aber auch in gesinterter Form vorliegen. Durch anschließendes Sintern verbindet sich die Keramikschicht formschlüssig mit dem Prothesenkörper.

Vorteilhafterweise werden der Suspension, in der das Granulat verteilt ist, Additive, wie z. B. Binder oder Dispergatoren zugefügt. Solche Additive verhindern das Absinken und Zusammenwachsen der einzelnen Granulatkörner und erhalten somit die durch das Auftragen entstandene poröse Struktur der Suspension bzw. des Schlickers.

Das Granulat selbst wird bevorzugt durch Sprühgranulierung oder Aufbaugranulierung hergestellt und nach dem Sintern durch Mörsern oder Mahlen nochmals zerkleinert um eine möglichst raue Oberfläche mit Spitzen und Kavitäten zu erhalten. Neben den bereits genannten Vorzügen des erfindungsgemäßen Verfahrens hat dies den Vorteil, im laufenden Herstellungsprozess der gesamten Keramik-Endoprothese integriert werden zu können.

Ein Ausführungsbeispiel der erfindungsgemäßen Keramik-Endoprothese ist in der Zeichnung beispielhaft dargestellt und anhand der folgenden Beschreibung näher erläutert. Es zeigen:
- Fig. 1: ein erstes Ausführungsbeispiel einer erfindungsgemäßen Keramik-Endoprothese in schematischer Darstellung;
- Fig. 2: ein erstes Ausführungsbeispiel zur Herstellung des Granulats durch ein Aufbaugranulierverfahren in schematische Darstellung;
- Fig. 3: ein zweites Ausführungsbeispiel zur Herstellung des Granulats durch ein Sprühgranulierverfahren in schematischer Darstellung;
- Fig. 4: eine schematische Darstellung der Bildung des Granulat;
- Fig. 5: eine Mikroskopaufnahme eines ersten erfindungsgemäßen Ausführungsbeispiels einer porösen Keramikschicht mit Mahlkugeln als Granulat und
- Fig. 6: eine Mikroskopaufnahme eines zweiten erfindungsgemäßen Ausführungsbeispiels einer porösen Keramikschicht mit in Sprühgranulierung hergestelltem Granulat.

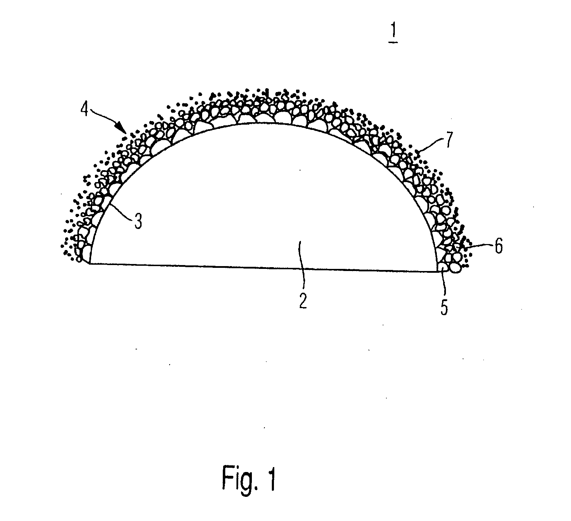

Fig. 1 zeigt eine Keramik-Endoprothese 1, hier in Form einer Hüft- oder Schultergelenkpfanne. Die Keramik-Endoprothese besteht aus einem Prothesenkörper 2, auf dessen dem Knochen zugewandter Oberfläche 3 eine poröse Keramikschicht 4 aufgebracht ist. Der Prothesenkörper 2 besteht typischerweise aus einer Aluminiumoxid- oder Zirkoniumdioxid-Keramik, die auch mit Yttriumoxid, Magnesiumoxid oder anderen Oxiden stabilisiert sein kann. Der Prothesenkörper 2 kann ebenso aus einer Dispersions-Keramik, wie z. B. Aluminiumoxid-verstärktem Zirkondioxid oder Zirkondioxid-verstärktem Aluminiumoxid bestehen, wobei das Zirkondioxid mit oder ohne Y₂O₃ stabilisiert sein kann. Ebenso kann die Keramikprothese aus Nichtoxidkeramik, z.B. Si₃N₄ oder SiC, bestehen.

Eine auf der knochenseitigen Oberfläche 3 des Prothesenkörpers 2 aufgebrachte poröse, raue Keramikschicht 4 kann aus einer oder auch mehreren Granulatschichten 5, 6 aufgebaut sein. Die abgebildete Keramik-Endoprothese 1 weist eine erste Granulatschicht 5 aus grobkörnigem Granulat auf. Das Granulat wird dabei mit einem Lösungsmittel wie z. B. Wasser, Additiven und einem Bindemittel wie z. B. Wachs, Polyurethan, Polylactane oder Polymerdispersionen, zu einer Suspension verarbeitet, deren Viskosität durch Additive eingestellt werden kann. Diese Suspension wird auf die knochenseitige Oberfläche 3 des Prothesenkörpers 2 aufgetragen und in einem Brennofen gesintert.

Der Brennvorgang dauert so lange an, bis das Granulat der ersten Granulatschicht 5 mit dem Keramikmaterial des Prothesenkörpers 2 versintert ist. Beim Sinterprozess verbinden sich einzelne Granulatkörnchen der ersten Granulatschicht 5 mit der Keramikstruktur des Prothesenkörpers 2 und bilden somit eine feste Verbindung miteinander aus. Beim Brennvorgang verdampfen die organischen Additive. Der Brennvorgang wird durch die Wahl z.B. der Sintertemperatur und der Prozessdauer so gesteuert, dass die poröse Struktur der Schicht erhalten bleibt.

Um noch feinere Strukturen an der Oberfläche der ersten Granulatschicht 5 aufzubringen, kann nach Belieben eine zweite Granulatschicht 6 mit z.B. feinkörnigem Granulat oder mit gemahlenem und/oder gemörsertem Granulat in gleicher Weise wie die erste Granulatschicht 5 aufgebracht und mit der ersten Granulatschicht 5 versintert werden. Durch das Mahlen und/oder Mörsern des hauptsächlich kugelförmigen Granulats entstehen teilkugelförmige Granulatfragmente mit unregelmäßiger Oberfläche, die durch Aneinanderlagern zu kleineren Poren und einer feiner strukturierten Oberfläche führen.

Eine Mikrorauheit der Oberfläche kann durch mechanische Verfahren, wie z.B. Sandstrahlen oder chemische Verfahren, beispielsweise durch Ätzprozesse mittel Verweilen der Endoprothese mit poröser Schicht in Salzschmelze erzeugt werden. Auch durch ein zusätzliches Beschichtungsverfahren, wie z.B. durch Kaltgasspritzen, kann eine Mikrorauheit in die Oberfläche eingebracht werden.

Der Rauheitswert Rₐ der porösen Keramikschicht 4 beträgt zwischen 20µm und 50µm, bevorzugt zwischen 30µm und 45µm und besonders bevorzugt 38µm.

Anschließend an die mindestens eine Granulatschicht kann zur Förderung des Knochenwachstums eine bioaktive Schicht 7 aufgebracht werden. Als bioaktives Material werden dabei insbesondere bevorzugt Hydroxylapatit (HAP) oder Calciumphosphat-basierte Stoffe wie Tri- oder Okta-Calciumphosphat (TCP, OPC) eingesetzt. Diese Materialien werden beispielsweise durch Kaltgasspritzen, Vakuumspritzen oder Plasmaspritzen auf die poröse Keramikschicht 4 aufgebracht. Zusätzlich können knochenwachstumsanregende Substanzen wie BMP (bone morphogenetic protein) aufgebracht werden. Das Knochenwachstum kann auch durch eine elektrochemisch aufgebrachte Schicht, z.B. Bonit, angeregt werden. Erfordert dies eine elektrisch leitende Implantatoberfläche 3, ist eine vorherige Beschichtung mit einer Ti- oder TiN-Schicht z.B. durch Physikalische Gasphasenabscheidung (PVD) möglich.

Eine so aufgebaute Keramik-Endoprothese 1 wird in eine Aushöhlung im Knochen entsprechend der Größe der Keramik-Endoprothese 1 leicht verpresst eingesetzt. Gefördert durch die bioaktive Schicht sowie die poröse Oberfläche der Prothese wird das Knochenwachstum angeregt, so dass nach einer Ausheilungsphase Knochengewebe in die poröse Oberflächenstruktur der Keramik-Endoprothese 1 eingewachsen ist.

Als Basismaterial für die Beschichtung können keramische Mahlkugeln, wie sie typischerweise in Keramikmühlen zum Zerkleinern von Keramikpulver verwendet werden, eingesetzt werden. Diese sind in verschiedenen Größen und Materialien kommerziell erhältlich. Mahlkugeln haben herkömmlicherweise eine glatte Oberfläche und bilden durch die Zwischenräume zwischen den einzelnen Kugeln Einwachskanäle für den Knochen. Eine rauere Oberfläche sowie eine höhere Strukturierung und damit größere Porosität der Keramikschicht 4 kann durch keramisches Granulat gebildet werden, das in einem Aufbaugranulierverfahren (10) oder Sprühgranulierverfahren 20 hergestellt wird.

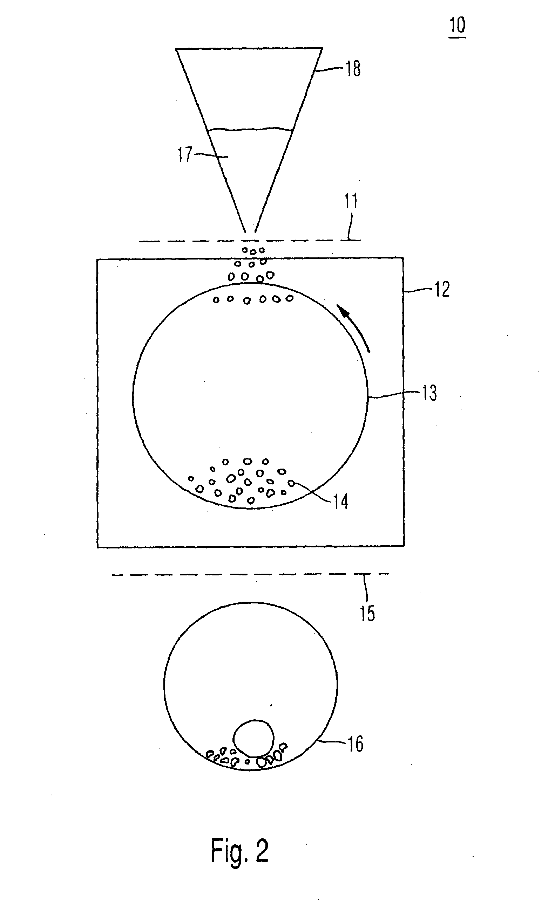

In einem Aufbaugranulierverfahren, wie in Fig. 2 dargestellt, wird dabei feines Keramikpulver mit Wasser und Additiven zu einer Suspension 17 verarbeitet und durch eine Düse 18 und/oder ein Einspritzsieb 11 in einen Brennofen 12 eingesprüht. Ein Drehrohr 13 im Brennofen 12 nimmt das Granulat auf und verhindert durch eine ständige Drehbewegung ein weiteres Agglomerieren, d. h. Zusammenbacken der einzelnen Granulatkörner 14. Mit einem Ausgangssieb 15 werden Granulatkörner 14, deren Größe höchstens gleich der Maschenweite ist, für die Weiterverwendung entnommen.

Nach dem Sintern des Granulats im Brennofen 12 wird dieses Granulat durch Mörsern oder Mahlen 16 nochmals zerkleinert. Dadurch wird die Oberfläche der Granulatkörner beschädigt und es entstehen zusätzliche Spitzen und Unebenheiten an deren Oberfläche. Beim Aufbringen auf den Prothesenkörper 2 entsteht somit eine Mikrorauigkeit mit vielen feinen Zwischenräumen und Kavitäten.

Beim Aufbaugranulieren 10 kann durch die Verwendung unterschiedlicher Düsen 18 bzw. von Einspritz- und/oder Ausgangsieben 11, 15 mit unterschiedlicher Maschenweite die Korngröße des Granulats einfach verändert werden. Es wird Granulat mit einer Korngröße von 0,01 mm bis 1mm, vorzugsweise mit 200 µm bis 500 µm, verwendet.

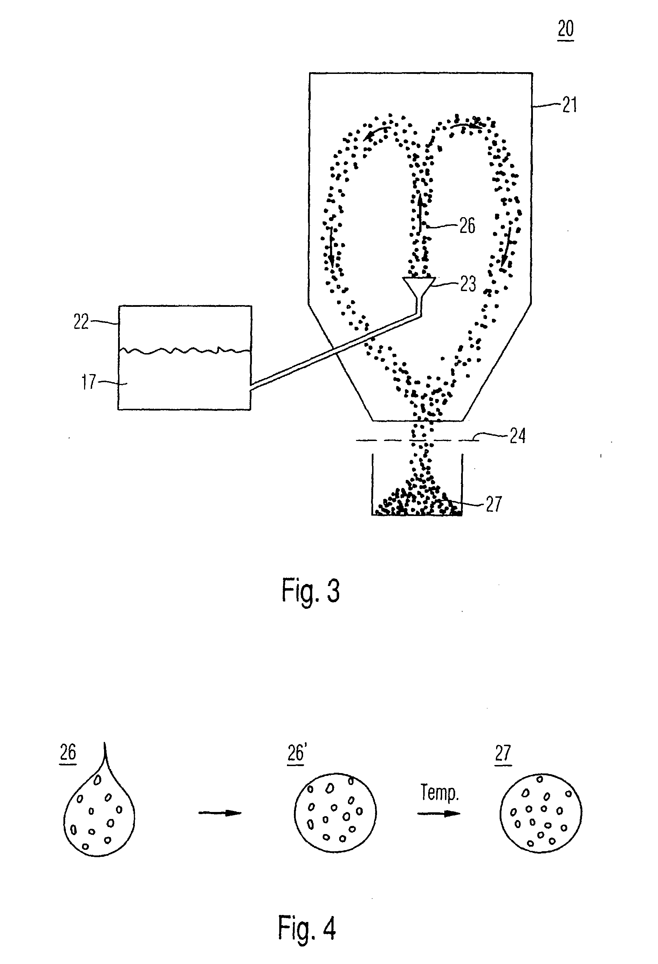

Bei einem Sprühgranulierverfahren 20, das in Fig. 3 gezeigt ist, wird eine Suspension 17 aus einem Vorratsbehälter 22 über eine Düse 23 nach oben in einen Sprühturm 21 eingesprüht. Während des Aufenthaltes im Sprühturm 21 bilden die Tröpfchen 26, wie in Fig. 4 dargestellt, eine Kugelform 26' aus. Durch die im Sprühturm 21 herrschende Temperatur verdampft die Flüssigkeit aus den kugelförmigen Tröpfchen 26' und die Kugelform verfestigt sich, so dass resultierend ein nahezu wasserfreies, kugelförmiges, poröses Granulat 27 vorliegt.

Durch ein Ausgangssieb 24 kann das Granulat 27 von Ausschuss (Grobkorn) getrennt und/oder bei Bedarf nach Größe sortiert werden. Granulatgröße, Granulatform und Porosität des Granulats sind über eine Variation der Düsenöffnung und Düsenform, der Schlickerzusammensetzung und der Sprühparameter, wie z.B. Sprühdruck und Temperatur im Sprühturm, steuerbar. Das erzeugte Granulat wird einem weiteren thermischen Prozess unterzogen um die poröse Granulatstruktur während der weiteren Verarbeitung zu erhalten. Das Granulat selbst weist eine Porosität von 30% bis 80%, bevorzugt von 40% bis 50% auf.

Das so hergestellte Granulat wird abermals mit Lösungsmitteln und Additiven zu einer Suspension verarbeitet und zu einem zähflüssigen Schlicker vermengt. Der Schlicker wird nun in einfacher Weise z. B. durch Aufpinseln oder mit Hilfe eines anderen Werkzeugs wie z. B. einem Spatel auf den Prothesenkörper 2 aufgebracht. Der Schlicker kann auch durch Aufsprühen und/oder durch Eintauchen des Prothesenkörpers 2 in den Schlicker und/oder durch ein Sol-Gel-Verfahren aufgebracht werden. Besteht die poröse Keramikschicht aus mehreren Granulatschichten 5, 6, können diese Schichten durch unterschiedliche Auftragungsverfahren appliziert werden. Die knochenseitige Oberfläche 3 des Prothesenkörpers kann zur besseren Anhaftung des Schlickers vor dem Aufbringen der ersten Granulatschicht 5 angeraut werden.

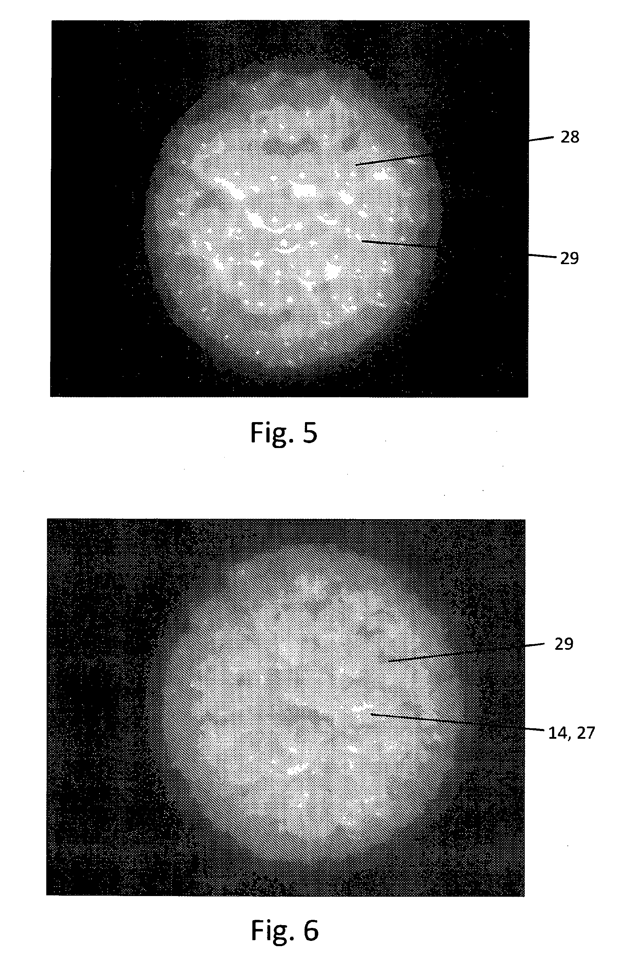

Fig. 5 zeigt eine Mikroskopaufnahme einer porösen Keramikschicht, in der Mahlkugeln 28 als Granulat verwendet sind. Die kugelige Form sowie die glatte Oberfläche der Mahlkugeln 28 sind deutlich zu erkennen. Zwischen den Mahlkugeln 28 sind große Kavitäten und Einwachskanäle 29 sichtbar.

Die poröse Keramikschicht in Fig. 6 umfasst Granulat 14, 27, das durch Aufbau- bzw. Sprühgranulierung hergestellt wurde. Die Oberfläche des Granulats 14, 27 weist eine höhere Rauigkeit als die Oberfläche der Mahlkugeln 28 auf, was durch eine geringer Lichtreflexion an der Oberfläche deutlich wird. Die Korngröße des Granulats 14, 27 ist geringer, sodass engere Einwachskanäle 29 und kleinere Kavitäten und Poren durch die Zwischenräume des Granulats 14, 27 gebildet werden.

Alle beschriebenen und/oder gezeichneten Merkmale können im Rahmen der Erfindung vorteilhaft miteinander kombiniert werden. Die Erfindung ist nicht auf die beschriebenen Ausführungsbeispiele beschränkt. So kann die Keramik-Endoprothese mit poröser Keramikschicht nicht nur als Hüft- bzw. Schultergelenkpfanne, sondern auch als Knieimplantat, Bandscheiben-Ersatzoder ähnliches eingesetzt werden.

## Patentansprüche

1. Keramik-Endoprothese zur Implantation in einen tierischen oder menschlichen Knochen, umfassend einen Prothesenkörper (2), der eine mit dem Knochen in Kontakt stehende knochenseitige Oberfläche (3) aufweist, auf die eine poröse, makrostrukturierte Keramikschicht (4) aufgebracht ist, die aus einem keramischen Granulat besteht,
**dadurch gekennzeichnet,**
dass das Granulat durch Mörsern oder Mahlen zerkleinert und teilkugelförmig ausgebildet ist.

2. Endoprothese nach Anspruch 1,
**dadurch gekennzeichnet,**
dass das Granulat durch Sintern stoff- und/oder formschlüssig mit dem Prothesenkörper (2) verbunden ist.

3. Endoprothese nach Anspruch 1 oder 2,
**dadurch gekennzeichnet,**
dass die poröse Keramikschicht (4) aus mehreren Granulat-Schichten (5,6) mit unterschiedlichen Korngrößen besteht.

4. Endoprothese nach einem der Ansprüche 1 bis 3,
**dadurch gekennzeichnet,**
dass auf die poröse Keramikschicht (4) eine Schicht aus einem bioaktiven Material (7) aufgebracht ist.

5. Endoprothese nach einem der Ansprüche 1 bis 4,
**dadurch gekennzeichnet,**
dass die poröse Keramikschicht (4) eine Oberfläche mit einer Mikrorauigkeit aufweist, die durch ein mechanisches Verfahren und/oder ein chemisches Verfahren und/oder ein Beschichtungsverfahren, eingebracht ist.

6. Endoprothese nach einem der Ansprüche 1 bis 5,
**dadurch gekennzeichnet,**
dass das Granulat aus Keramikkügelchen, insbesondere aus Mahlkugeln, besteht.

7. Endoprothese nach einem der Ansprüche 1 bis 5,
**dadurch gekennzeichnet,**
dass das Granulat durch Sprühgranulierung oder Aufbaugranulierung hergestellt ist.

8. Endoprothese nach einem der Ansprüche 1 bis 7
**dadurch gekennzeichnet,**
dass das Granulat eine mittlere Korngröße zwischen 0,01 mm bis 1 mm, bevorzugt eine Korngröße von 200 µm bis 500 µm, aufweist.

9. Verfahren zur Herstellung einer Keramik-Endoprothese zur Implantation in einen tierischen oder menschlichen Knochen, umfassend einen Prothesenkörper (2), der eine mit dem Knochen in Kontakt stehende knochenseitige Oberfläche (3) aufweist, auf die eine poröse, makrostrukturierte Keramikschicht (4) aufgebracht wird, wobei die poröse Keramikschicht (4) aus keramischem Granulat gebildet wird,
**dadurch gekennzeichnet,**
dass das Granulat durch Mörsern oder Mahlen (16) nochmals zerkleinert wird und teilkugelförmig ausgebildet ist.

10. Verfahren nach Anspruch 9,
**dadurch gekennzeichnet,**
dass das Granulat als Suspension zu einem Schlicker vermengt auf den Prothesenkörper (2) aufgebracht und durch Sintern stoff- und/oder formschlüssig mit dem Prothesenkörper (2) verbunden wird.

11. Verfahren nach Anspruch 9 oder 10,
**dadurch gekennzeichnet,**
dass mehrere Granulat-Schichten (5, 6) mit unterschiedlichen Korngrößen auf den Prothesenkörper (2) aufgebracht werden.

12. Verfahren nach einem der Ansprüche 9 bis 11,
**dadurch gekennzeichnet,**
dass auf die poröse Keramikschicht (4) eine Schicht aus einem bioaktiven Material (7) aufgebracht wird.

13. Verfahren nach einem der Ansprüche 9 bis 12,
**dadurch gekennzeichnet,**
dass die poröse Keramikschicht eine Oberfläche mit einer Mikrorauigkeit aufweist, die durch ein mechanisches und/oder ein chemisches Verfahren und/oder ein Beschichtungsverfahren eingebracht ist.

14. Verfahren nach Anspruch 10,
**dadurch gekennzeichnet,**
dass der Suspension Additive, wie z.B. Binder und Dispergatoren, beigemischt werden.

15. Verfahren nach einem der Ansprüche 9 bis 14,
**dadurch gekennzeichnet,**
dass das Granulat durch Sprühgranulierung (10) oder durch Aufbaugranulierung hergestellt wird.

16. Verfahren nach einem der Ansprüche 9 bis 14,
**dadurch gekennzeichnet,**
dass Mahlkugeln als Granulat verwendet werden.

17. Verfahren nach einem der Ansprüche 9 bis 16,
**dadurch gekennzeichnet,**
dass das Granulat eine mittlere Korngröße zwischen 0,01 mm bis 1 mm, bevorzugt eine Korngröße von 200 µm bis 500 µm, aufweist.

## Claims

1. Ceramic endoprosthesis for implantation in an animal bone or a human bone, comprising a prosthesis base (2) which has, on the side towards the bone, a surface (3) that is in contact with the bone and onto which a porous, macrostructured ceramic layer (4) composed of a ceramic granulate has been applied,
**characterized in that**
the granulate has been comminuted by trituration or milling and has the shape of part of a sphere.

2. Endoprosthesis according to Claim 1,
**characterized in that**
the granulate has been coherently bonded and/or interlock-bonded to the prosthesis base (2).

3. Endoprosthesis according to Claim 1 or 2,
**characterized in that**
the porous ceramic layer (4) is composed of a plurality of granulate layers (5, 6) with different grain sizes.

4. Endoprosthesis according to any of Claims 1 to 3,
**characterized in that**
a layer made of a bioactive material (7) has been applied to the porous ceramic layer (4).

5. Endoprosthesis according to any of Claims 1 to 4,
**characterized in that**
the porous ceramic layer (4) has a surface with microroughness introduced via a mechanical process and/or a chemical process and/or a coating process.

6. Endoprosthesis according to any of Claims 1 to 5,
**characterized in that**
the granulate is composed of ceramic beads, in particular of milling beads.

7. Endoprosthesis according to any of Claims 1 to 5,
**characterized in that**
the granulate has been produced by spray granulation or agglomerative granulation.

8. Endoprosthesis according to any of Claims 1 to 7,
**characterized in that**
the average grain size of the granulate is from 0.01 mm to 1 mm, preferably from 200 µm to 500 µm.

9. Process for the production of a ceramic endoprosthesis for implantation in an animal bone or a human bone, comprising a prosthesis base (2) which has, on the side towards the bone, a surface (3) that is in contact with the bone and onto which a porous, macrostructured ceramic layer (4) is applied, where the porous ceramic layer (4) is formed from ceramic granulate,
**characterized in that**
the granulate is further comminuted by trituration or milling (16) and has the shape of part of a sphere.

10. Process according to Claim 9,
**characterized in that**
the granulate applied to the prosthesis base (2) takes the form of suspension mixed to give a slurry, and is coherently bonded and/or interlock-bonded to the prosthesis base (2) by sintering.

11. Process according to Claim 9 or 10,
**characterized in that**
a plurality of granulate layers (5, 6) with different grain sizes are applied to the prosthesis base (2).

12. Process according to any of Claims 9 to 11,
**characterized in that**
a layer made of a bioactive material (7) is applied to the porous ceramic layer (4).

13. Process according to any of Claims 9 to 12,
**characterized in that**
the porous ceramic layer has a surface with microroughness introduced via a mechanical process and/or a chemical process and/or a coating process.

14. Process according to Claim 10,
**characterized in that**
additives, e.g. binders and dispersing agents, are admixed with the suspension.

15. Process according to any of Claims 9 to 14,
**characterized in that**
the granulate is produced by spray granulation (10) or agglomerative granulation.

16. Process according to any of Claims 9 to 14,
**characterized in that**
milling beads are used as granulate.

17. Process according to any of Claims 9 to 16,
**characterized in that**
the average grain size of the granulate is from 0.01 mm to 1 mm, preferably from 200 µm to 500 µm.

## Revendications

1. Endoprothèse en céramique destinée à être implantée dans un os animal ou humain, comprenant un corps de prothèse (2) qui présente une surface (3) située du côté de l'os et en contact avec l'os, et sur laquelle est appliquée une couche poreuse de céramique (4), macro-structurée et constituée d'un granulat céramique,
**caractérisée**
en ce que le granulat est fractionné au broyeur à mortier ou au broyeur à moulin, et se présente sous forme de billes partielles fractionnées.

2. Endoprothèse selon la revendication 1,
**caractérisée**
en ce que le granulat est relié, par frittage, au corps de prothèse (2), en formant avec celui-ci une liaison par continuité de matière et/ou par complémentarité de formes.

3. Endoprothèse selon la revendication 1 ou la revendication 2,
**caractérisée**
en ce que la couche poreuse de céramique (4) est constituée de plusieurs couches de granulat (5, 6) présentant des grosseurs de grain différentes.

4. Endoprothèse selon l'une des revendications 1 à 3,
**caractérisée**
en ce que sur la couche poreuse de céramique (4) est appliquée une couche en un matériau bioactif (7).

5. Endoprothèse selon l'une des revendications 1 à 4,
**caractérisée**
en ce que la couche poreuse de céramique (4) présente une surface avec une microrugosité qui est produite par un procédé mécanique et/ou un procédé chimique et/ou un procédé de revêtement.

6. Endoprothèse selon l'une des revendications 1 à 5,
**caractérisée**
en ce que le granulat est constitué de petites billes de céramique, notamment de billes de broyage au moulin.

7. Endoprothèse selon l'une des revendications 1 à 5,
**caractérisée**
en ce que le granulat est fabriqué par granulation par pulvérisation ou granulation par agglomération.

8. Endoprothèse selon l'une des revendications 1 à 7,
**caractérisée**
en ce que le granulat présente une grosseur de grain moyenne entre 0,01 mm et 1 mm, de préférence une grosseur de grain de 200 µm à 500 µm.

9. Procédé de fabrication d'une endoprothèse en céramique destinée à être implantée dans un os animal ou humain, comprenant un corps de prothèse (2) qui présente une surface (3) située du côté de l'os et en contact avec l'os, et sur laquelle on applique une couche poreuse de céramique (4), macro-structurée, la couche poreuse de céramique (4) étant constituée d'un granulat céramique,
**caractérisé** en ce que le granulat est fractionné une nouvelle fois au broyeur à mortier ou au broyeur à moulin (16), et se présente sous forme de billes partielles fractionnées.

10. Procédé selon la revendication 9,
**caractérisé** en ce que le granulat est appliqué sur le corps de prothèse (2), en tant que suspension en étant additionné à une barbotine, et est relié par frittage, au corps de prothèse (2), en formant avec celui-ci une liaison par continuité de matière et/ou par complémentarité de formes.

11. Procédé selon la revendication 9 ou la revendication 10,
**caractérisé** en ce que l'on applique plusieurs couches de granulat (5, 6) présentant des grosseurs de grain différentes, sur le corps de prothèse (2).

12. Procédé selon l'une des revendications 9 à 11,
**caractérisé** en ce que l'on applique une couche en un matériau bioactif (7) sur la couche poreuse de céramique (4).

13. Procédé selon l'une des revendications 9 à 12,
**caractérisé** en ce que la couche poreuse de céramique (4) présente une surface avec une microrugosité qui est produite par un procédé mécanique et/ou chimique et/ou un procédé de revêtement.

14. Procédé selon la revendication 10,
**caractérisé** en ce que l'on mélange des additifs, comme par exemple des liants et des dispersants, à la suspension.

15. Procédé selon l'une des revendications 9 à 14,
**caractérisé** en ce que l'on fabrique le granulat par granulation par pulvérisation (10) ou granulation par agglomération.

16. Procédé selon l'une des revendications 9 à 14,
**caractérisé** en ce que l'on utilise des billes de broyage au moulin en tant que granulat.

17. Procédé selon l'une des revendications 9 à 16,
**caractérisé** en ce que le granulat présente une grosseur de grain moyenne entre 0,01 mm à 1 mm, de préférence une grosseur de grain de 200 µm à 500 µm.
